Europäisches Patentamt

(19) **European Patent Office**

**Office européen des brevets**

(11) Numéro de publication: **0 149 367**
**A1**

(12) **DEMANDE DE BREVET EUROPEEN**

(21) Numéro de dépôt: **84400100.8**

(22) Date de dépôt: **18.01.84**

(51) Int. Cl.⁴: **A 61 D 7/00**
**A 61 F 5/47**

(43) Date de publication de la demande:
**24.07.85 Bulletin 85/30**

(84) Etats contractants désignés:
**AT BE CH DE GB IT LI LU NL SE**

(71) Demandeur: **Société Civile française dite "Européenne de Recherche d'Instrumentations Médicales S E R I M E D"**
**154, rue du Faubourg Saint Denis**
**F-75010 Paris(FR)**

(72) Inventeur: **Basuyaux, Michel**
**219, boulevard de la Liberté**
**F-59000 Lille (Nord)(FR)**

(74) Mandataire: **Rataboul, Michel**
**Cabinet Michel Rataboul 69, rue de Richelieu**
**F-75002 Paris(FR)**

(54) **Stérilet endo-utérin pour les animaux tels que les chiennes.**

(57) Le stérilet selon l'invention est du type comprenant une tige centrale 1 et un dispositif de maintien en place de ladite tige 1 à l'intérieur de la cavité utérine d'un sujet, dispositif composé de deux bras élastiques 4-5, symétriques par rapport à la tige 1 dont ils sont solidaires en son sommet 1a.

Il est caractérisé en ce que les bras occupent naturellement une position dans laquelle ils divergent au-delà du sommet 1a en faisant entre eux un angle α de valeur au moins égale à celle de l'angle que font généralement entre elles deux cornes B-C Que présente l'utérus A des femelles d'une espèce animale donnée, et tout particulièrement l'espèce canine, afin que les bras 4-5 puissent être engagés dans les cornes B-C et y être maintenus par l'effet d'écartement que produit leur élasticité naturelle.

**FIG.1**

## STERILET ENDO-UTERIN POUR LES ANIMAUX TELS QUE LES CHIENNES

Parmi les méthodes de contraception humaine connues, se trouve celle qui consiste à placer un stérilet dans la cavité utérine.

Il existe de nombreuses variantes concernant le problème du maintien en place du stérilet sans provoquer ni blessure ni gêne.

Mais les stérilets connus jusqu'à ce jour sont le plus souvent adaptés à l'utérus de la femme dont les dimensions et les formes sont telles que le dispositif de maintien en place du stérilet prend toujours appui sur les parois endo-utérines.

Jusqu'à maintenant, il n'a été possible d'utiliser les principes connus des stérilets utilisés chez la femme pour les appliquer, en les adaptant au besoin quelque peu, à des espèces animales dont on veut contrôler les naissances que pour des espèces dont l'utérus est de même type que celui de la femme. Pour les autres espèces, cette adaptation a été impossible notamment pour les espèces canine et chevaline.

En effet, le système génital de ces espèces est assez différent de celui de la femme et aucune transposition simple ne s'est révélée satisfaisante.

C'est pourquoi on se contente jusqu'à maintenant de méthodes soit peu sûres telles que les répulsifs, les contraceptifs hormonaux oraux ou les contraceptifs hormonaux injectables, soit radicales, dangereuses et onéreuses telles que l'ovariéctomie (abaltion des ovaires) ou la ligature des cornes utérines.

Il serait du plus grand intérêt aussi bien pour les propriétaires d'animaux que pour les animaux eux-mêmes de pouvoir utiliser un stérilet car, comme on le sait, il s'agit d'une méthode simple et peu onéreuse, d'une réversibilité parfaite (l'enlèvement du stérilet est très facile en cas de grossess désirée), qui ne présente aucun inconvénient au regard de la santé ou du comportement du sujet et, enfin, qui présente une fiabilité excellente.

Pour illustrer l'état de la technique, on peut signaler, en particulier, le brevet US-A-3.507.274 qui décrit un stérilet ayant des bras divergents dont les faces extérieures 28 des parties développées 24 doivent être au contact des parois 29 de l'intérieur 30 de la cavité utérine A, sous les canaux ou ouvertures D des trompes (page 1, colonne 2, lignes 55 à 59).                    .../...

On connaît également le brevet GB-A-2.079.158 qui concerne bien un stérilet destiné aux femelles de certaines espèces animales, mais il ne contient aucun enseignement à propos du maintien en place du stérilet par les cornes utérines et décrit, au contraire, la mise en place du stérilet dans la cavité utérine elle-même, l'ensemble du stérilet ayant une élasticité suffisante pour permettre le pliage de ses lobes (et non de bras) et leur déploiement élastique naturel (page 2, lignes 31 à 37).

Le brevet FR-A-78/16.160 décrit, lui aussi, un stérilet dont les bras doivent prendre appui contre la paroi de l'utérus, ainsi que cela est précisé page 1, ligne 17 et lignes 19 à 22. Le stérilet conforme à l'invention est destiné à certaines espèces animales et ne pourrait être utilisé pour les femmes dont l'utérus est démuni de cornes, alors que le stérilet décrit dans ce brevet n'est usilisable qu'en médecine (et non en art vétérinaire), ainsi que cela résulte des indications telles que les mots : "médecine" page 1, ligne 2, "confort de la patiente" page 1, ligne 10, "médecin" page 2, ligne 27 etc.

En outre, il est clairement établi que ce stérilet ne présente pas des bras divergents au-delà du sommet de la tige.

La présente invention permet au contraire de réaliser un stérilet destiné aux femelles des espèces animales dont la cavité utérine est différente de celle de l'espèce humaine, ce qui est le cas, tout particulièrement, des chiennes.

Un stérilet conforme à l'invention est du type comprenant une tige centrale et un dispositif de maintien en place de ladite tige à l'intérieur de la cavité utérine d'un sujet, dispositif composé de deux bras élastiques qui sont symétriques par rapport à la tige dont ils sont solidaires en son sommet, et qui occupent naturellement une position dans laquelle ils divergent au-delà du sommet de la tige en faisant entre eux un angle, caractérisé en ce que le sommet de la tige doit se situer au voisinage du fond de la cavité utérine, les bras devant se placer au-delà de cette cavité, dans deux cornes utérines que présente l'utérus des femelles d'une espèce animale donnée, et tout particulièrement l'espèce canine, l'angle des bras étant supérieur à celui que font entre elles lesdites cornes utérines.

Selon d'autres caractéristiques de l'invention :

- les bras portent à leur extrémité des reliefs extérieurs à surface courbe non blessante et, de préférence, donnant à l'extrémité de chaque bras une forme hémi-ovoïde;

.../...

- le stérilet est fait en une matière synthétique, telle que le polyéthylène, contenant des parcelles de cuivre dispersées;

- le stérilet est fait en une matière synthétique, telle que le polyéthylène, contenant au moins un sel métallique tel que le sulfate de baryum.

L'invention sera mieux comprise par la description détaillée ci-après faite en référence au dessin annexé. Bien entendu, la description et le dessin ne sont donnés qu'à titre d'exemple indicatif et non limitatif.

La figure 1 est une vue schématique de profil d'un stérilet conforme à l'invention.

Les figures 2, 3 et 4 sont des vues schématiques montrant la mise en place d'un stérilet conforme à l'invention dans la cavité utérine d'une chienne.

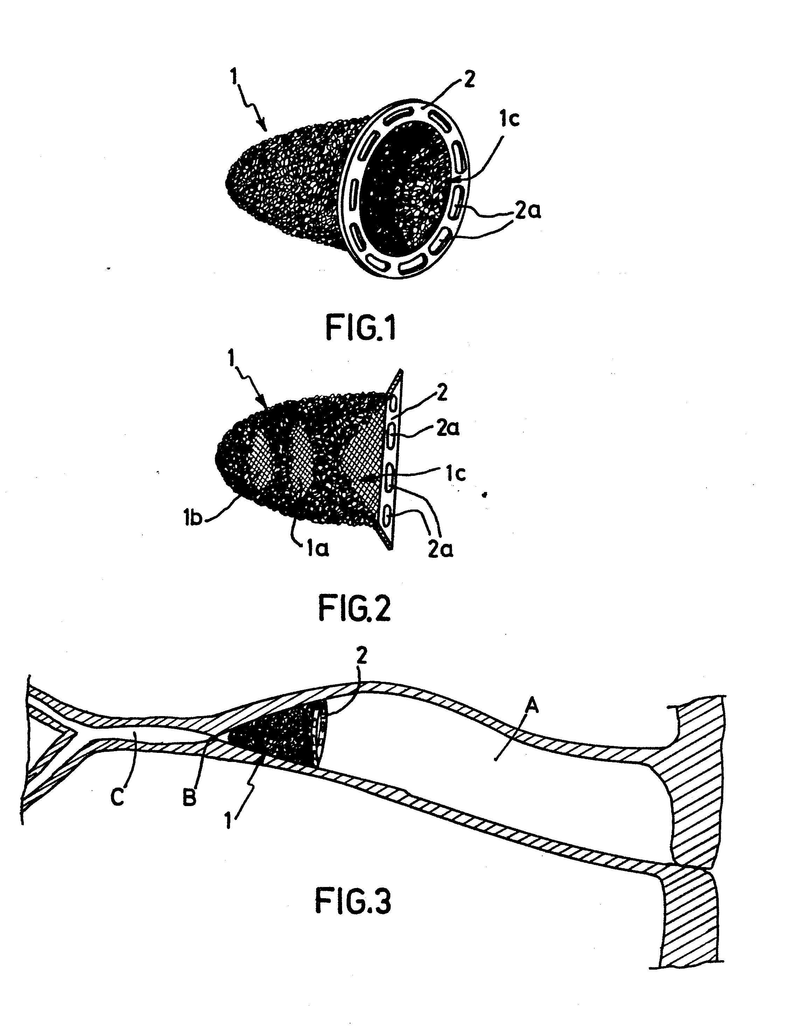

En se reportant à la figure 1, on voit un stérilet conforme à l'invention du type comprenant une tige centrale 1 sur laquelle est enroulé, ainsi que cela est connu, un fil de cuivre 2 et qui est munie à sa base d'un passage transversal pour deux fils 3 destinés au repérage et à l'extraction du stérilet comme cela est bien connu avec les stérilets destinés aux femmes.

Le sommet 1a de la tige 1 est solidaire de deux bras 4 et 5, l'ensemble de la tige 1 et des deux bras 4 et 5 étant avantageusement obtenu par moulage en une seule pièce d'une matière synthétique, afin que ces deux bras 4 et 5 soient élastiquement déformables tout en ayant suffisamment de tenue et de "mémoire".

En effet, il faut que les bras 4 et 5 occupent naturellement une position dans laquelle ils divergent, au-delà du sommet 1a, en faisant entre eux un angle $\alpha$.

Les bras 4 et 5 sont susceptibles d'être pliés l'un vers l'autre, à l'encontre de leur élasticité, afin qu'ils se placent dans le prolongement de la tige 1. Pour qu'ils s'appliquent bien l'un contre l'autre, ils se font face par une surface plane.

De la sorte, ils peuvent être tous deux engagés avec la tige 1 dans un tube amovible de faible diamètre 6 comme cela est représenté sur les figures 2 et 3.

Le stérilet qui vient d'être décrit est du type endo-utérin c'est-à-dire qu'il doit être placé entièrement à l'intérieur de la cavité utérine A du sujet, le sommet 1a devant être situé au fond de cette cavité A.

.../...

Il est destiné aux femelles d'une espèce animale dont l'utérus A présente deux cornes B et C.

Celles-ci divergent en formant un angle virtuel dont la valeur est suffisamment bien connue pour que l'on se contente d'une bonne approximation, au moins pour une race donnée.

L'angle $\alpha$ est choisi pour être au moins égal à cet angle naturel des cornes utérines afin que les bras 4 et 5 puissent être engagés chacun dans l'une des deux cornes B et C, c'est-à-dire au-delà de la cavité utérine A proprement dite.

Il y sont maintenus par l'effet d'écartement que produit leur élasticité naturelle du fait qu'ils ne pourraient s'extraire accidentellement et provoquer ainsi l'expulsion de la tige 1 que s'ils étaient rapprochés l'un de l'autre selon un angle sensiblement égal à celui des cornes B et C.

En se reportant maintenant aux figures 2 à 4 on va décrire la mise en place d'un stérilet conforme à celui de la figure 1.

Les bras 4 et 5 sont rabattus l'un contre l'autre et l'ensemble de la tige 1, des fils 3 et des bras 4 et 5 est engagé dans le tube 6.

Par l'autre extrémité du tube 6, on engage un poussoir 7 et le tout est engagé dans une canule 8 du type rectoscope humain de dimensions voulues.

La canule 8 est engagée dans la vulve et est poussée jusqu'à ce que son extrémité butte dans le fond du vagin D à l'entrée de l'utérus A, c'est-à-dire en regard de l'orifice externe du col de cet utérus A.

On pousse alors le tube 6 pour qu'il passe le col et s'étende à l'intérieur de l'utérus A ce qui est représenté sur la figure 2.

On agit alors sur le poussoir 7 tout en retenant ou en tirant le tube 6 de sorte que les bras 4 et 5 du stérilet échappent à ce tube 6 qui, en ne les pinçant plus, laisse agir l'élasticité naturelle de la matière qui les constitue pour provoquer leur écartement.

En poussant le corps 1, on s'assure qu'il est bien entièrement placé dans la cavité utérine A, le sommet 1a devant alors se trouver au voisnage du fond de la cavité A, ce qui correspond à la mise en place correcte des bras 4 et 5 car, bien entendu, les dimensions relatives du corps 1 et des bras 4 et 5 sont établies en fonction du sujet, c'est-à-dire en fonction de la profondeur estimée pour une race donnée. On peut, ainsi par exemple, disposer de trois formats de stérilet correspondant respectivement aux chiennes de races "petites", "moyennes" et "grandes".

La figure 3 représente une phase intermédiaire de la mise en place et qui correspond au début de la pénétration des bras 4 et 5 dans les cornes B et C, la tige 1 du stérilet n'ayant pas encore été poussée suffisamment pour que son sommet 1a se trouve au fond de la cavité utérine A.

Lorsque cela est obtenu, ainsi qu'on l'a précisé plus haut, on retire à la fois le tube 6, le poussoir 7 et la canule 8. Le stérilet se trouve mis en place ainsi que cela est représenté sur la figure 4.

On voit bien, sur cette figure 4, que le stérilet est placé beaucoup plus haut dans l'utérus que les stérilets connus, pour que son sommet 1a soit situé au déboucher des cornes B et C, où son action contraceptive doit déjà s'exercer.

La base 1b de la tige 1 est constituée comme celle des stérilets utilisés pour les femmes c'est-à-dire qu'elle a une forme non blessante et propice, simultanément, à la ligature des fils 3 par lesquels on peut procéder à l'extraction du stérilet, ainsi que cela est connu en soi.

Afin de faciliter la mise en place du stérilet, c'est-à-dire la bonne introduction des bras 4 et 5 dans les cornes B et C, l'extrémité de ces bras 4 et 5 est munie de reliefs extérieurs respectivement 4a et 5a à surface courbe non blessante.

Ces reliefs forment des surépaisseurs qui jouent en quelque sorte le rôle de patins, glissant sans effort contre les parois des cornes B et C et s'opposant ainsi à toute perforation due à l'extrémité des bras 4 et 5.

Pour être sûr que ces reliefs jouent leur rôle quelle que soit la morphologie précise d'un sujet donné, il est bon que ces reliefs 4a et 5a aient un profil correspondant à une surface de révolution mais comme il est aussi important que les bras 4 et 5 puissent être placés l'un contre l'autre dans le tube 6 de plus petit diamètre possible, il est bon que le volume résultant de cette surface de révolution provienne de la combinaison des deux reliefs 4a et 5a. Chacun doit alors avoir la forme d'un hémi-ovoïde ou analogue (hémisphère, etc.).

La tige 1 et les bras 4 et 5 sont avantageusement moulés en matière synthétique telle que le polyéthylène et le fil de cuivre 2 est enroulé autour de la tige 1 comme cela est connu en soi.

Mais on peut aussi mélanger au polyéthylène, avant moulage, des parcelles de cuivre qui seront convenablement dispersées ainsi que, le cas échéant, des particules d'un sel métallique tel que le sulfate de baryum.

Cela renforce le pouvoir contraceptif par un processus d'ionisation non seulement vers les parois de la cavité utérine A, mais aussi vers les parois des cornes B et C, grâce à l'emplacement que le stérilet occupe conformément à l'invention et grâce, donc, à sa structure particulière.

*\*\**

# R E V E N D I C A T I O N S

1- Stérilet du type comprenant une tige centrale (1) et un dispositif de maintien en place de ladite tige (1) à l'intérieur de la cavité utérine (A) d'un sujet, dispositif composé de deux bras élastiques (4-5) qui sont symétriques par rapport à la tige (1) dont ils sont solidaires en son sommet (1a) et qui occupent naturellement une position dans laquelle ils divergent au-delà du sommet (1a) de la tige (1) en faisant entre eux un angle (α), caractérisé en ce que le sommet (1a) de la tige (1) doit se situer au voisinage du fond de la cavité utérine (A), les bras (4 et 5) devant se placer au-delà de cette cavité (A), dans deux cornes utérines (B et C) que présente l'utérus des femelles d'une espèce animale donnée, et tout particulièrement l'espèce canine, l'angle (α) des bras (4 et 5) étant supérieur à celui que font entre elles lesdites cornes utérines (B et C).

2- Stérilet selon la revendication 1, caractérisé en ce que les bras (4 et 5) portent à leur extrémité des reliefs extérieurs (4a et 5a) à surface courbe non blessante et, de préférence, donnant à l'extrémité de chaque bras une forme hémi-ovoïde.

3- Stérilet selon la revendication 1, caractérisé en ce qu'il est fait en une matière synthétique, telle que le polyéthylène, contenant des parcelles de cuivre dispersées.

4- Stérilet selon la revendication 1, caractérisé en ce qu'il est fait en une matière synthétique, telle que le polyéthylène, contenant au moins un sel métallique tel que le sulfate de baryum.

*****

FIG.1

FIG.2

FIG.3

Office européen
des brevets

## RAPPORT DE RECHERCHE EUROPEENNE

EP 84 40 0100

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| D,A | US-A-3 507 274 (SOICHET) <br> * Colonne 2, lignes 49-59; colonne 3, lignes 40-50; figures 1-3 * | 1,2 | A 61 D 7/00 <br> A 61 F 5/47 |
| | --- | | |
| D,A | GB-A-2 079 158 (MILLAR) <br> * Page 2, lignes 31-37; figures 1-4 * | 1 | |
| D,A | * Page 1, ligne 126 - page 2, ligne 6 * | 3 | |
| | --- | | |
| D,A | FR-A-2 427 088 (THOUVENIN) <br> * Page 1, lignes 23-30; page 2, lignes 20-24; page 4, revendication 4; figures 1-3 * | 3,4 | |
| | --- | | |
| E | FR-A-2 536 987 (BASUYAUX) <br> * En entier * | 1-4 | |
| | ----- | | |

**DOMAINES TECHNIQUES RECHERCHES (Int. Cl. ³)**

A 61 D
A 61 F

Le présent rapport de recherche a été établi pour toutes les revendications

| Lieu de la recherche <br> LA HAYE | Date d'achèvement de la recherche <br> 27-08-1984 | Examinateur <br> KNAUER F.E. |
|---|---|---|

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

\& : membre de la même famille, document correspondant

OEB Form 1503. 03.82